# EUROPEAN PATENT SPECIFICATION

(11) **EP 0 792 130 B1**
(45) Date of publication and mention of the grant of the patent: **19.07.2000**
(21) Application number: 95939251.5
(22) Date of filing: 10.11.1995
(51) Int. Cl.: A61F 5/01, A61H 1/00

(54) **EXTERNAL STRAIGHTENER FOR COLONOSCOPY**
EXTERNE DARMAUSRICHTUNGSVORRICHTUNG FÜR DIE KOLONOSKOPIE
DISPOSITIF REDRESSEUR EXTERNE DESTINE A UNE COLOSCOPIE

(30) Priority: 15.11.1994 IT CE940005
(43) Date of publication of application: 03.09.1997
(73) Proprietor: Advantech S.R.L., 20131 Milano (IT)
(72) Inventor: PATERNUOSTO, Mario, Immacolato, I-81100 Caserta (IT)
(74) Representative: Zavattoni, Maria Chiara
(86) International application number: EP9504420
(87) International publication number: WO9614811

(56) References cited:
- US-A- 3 111 317
- US-A- 4 969 222

## Description

Colonoscopy and pancolonoscopy are endoscopic investigations that are carried out with fibre optic endoscopes (fiberscopes) or with video endoscopes to explore the colon (the final portion of the intestine that goes from the caecum to the anus). This investigation, which poses a fair amount of difficulty both for the operator and for the patient, is performed by passing the fiberscope into the anus and advancing it under manual pressure by the operator as far as the caecum. This advancement is not easy because the intestine has curves and flexures that make it problematical, with considerable lengthening of the time required for the examination and above all with considerable discomfort for the patient undergoing it. To overcome such drawbacks the endoscopist must therefore rely on administration of drugs that sedate the patient and on external manoeuvres, usually performed by hand by an assistant, to compress the abdomen at various levels. These manoeuvres, however, are often ineffective.

Colonoscopy differs from other endoscopic examinations in that it can only partially follow pre-established plans for its performance and requires different manoeuvres according to the situations encountered, The sigma or sigmoid colon and the transverse colon have mesenteries, unlike the ascending and descending colon which are retroperitoneal. The presence of more or less long mesenteries facilitates a greatly feared trap of the colonoscopic examination: the so-called loop. In the event of a loop in the intestine, the patient experiences pain and the endoscope will not advance; indeed when pressure is applied the instrument undergoes a paradoxical backward movement.

In the sigmoid the shape of the pelvis and the sacral promontory pushes the tip of the apparatus towards the anterior abdominal wall; to reach the descending colon, however, the probe must turn posteriorly. To perform this manoeuvre the probe is turned clockwise. During this stage the endoscopist often asks for help from an assistant for targeted compression on the sigmoid in order to avoid loop formation. The problem with this compression is that it is not always targeted or even constant and uniform in that the action exerted inevitably depends on the patient's anatomical conformation (the action is more difficult and requires greater pressure for a large-bellied patient) and in that as time passes the pressure applied by the assistant suffers from the effects of tiredness.

It is also known that in the event of poor control of the endoscopic probe, the consequences can be catastrophic in patients at risk. Moreover, endoscopy is a difficult examination to learn and to teach. A further drawback is that it is difficult to repeat if it has been excessively stressful the first time. It should be noted in this respect that the 5 examination cannot be carried out under anaesthesia because to remove the pain symptom means to lose a sign of danger; sometimes pharmacological sedation can be employed but this is not always tolerated.

An internal straightener has been used in an attempt to at least partially overcome the serious drawbacks mentioned above, not without the occurrence of some complications, but its use has not encountered much favour.

According to the inventor's studies, pancolonoscopy could be simplified enormously if the following conditions were achieved:
1) try to "verticalize" the probe in the sigmoid as much as possible;
2) keep the probe as "vertical" as possible in the sigmoid throughout the course of the examination (the sigmoid together with the transverse colon is the most mobile portion of the colon in that it is connected to the wall by a more or less long tie) and avoid excessive mobility ("waving") of the sigmoid during insertion;
3) try to smooth the splenic flexure;
4) try to horizontalize the transverse colon;
5) allow the examination to be formed using the shortening technique to a minimal extent, this being a technique that shortens the colon with traction on the probe (which telescopes the intestine);
6) have the possibility of planning operating manoeuvres to facilitate the position of the probe;
7) not reach the hepatic flexure with excessive insertion of the probe in that the movements of the tip would become sluggish, reducing manoeuvrability.

The inventor's aim was to achieve one or more of said conditions.

The aims have been reached with an external straightener for colonoscopy as disclosed in claim 1. Further characteristics of the device are disclosed in the subsequent claims.

The new straightener is an article generally made of rubber, PVC, natural latex or similar relatively heavy material comprising an ovoid-shaped body with two opposite ends on a major axis, from which body, near one of the ends, a roundish protuberance projects. What is meant by "ovoid shape" in this text is a convex curved shape, which, besides being an egg shape, can be shaped like an ellipsoid, a rugby ball or the like. The length of the major axis of the body is of the order of 50-400 mm, preferably 300 mm, and more preferably 292 mm. The cross section of the body, about halfway along the major axis, is substantially circular with a diameter ranging between 50-250 mm, preferably about 200 mm, and more preferably 193 mm. The maximum distance of the surface of the protuberance from the long axis of the body is preferably 107 mm. The protuberance extends more or less along an axis c that is inclined with respect to the longitudinal axis of the body by d=about 38° and passes through the centre of the body. The protuberance has a major axis of about 140 mm and a minor axis of about 110 mm. The straightener must have a fairly considerable weight, a preferred weight being about 3.5 kg.

The method for use of the device will be described below with reference to the figures. The device makes it possible to achieve the aims listed above. In particular it makes it possible to exploit the patient's own weight in the various positions of use; thus the pressure exerted on the patient's belly is correlated to the patient him/herself and is relatively constant during the operation, as is appropriate for good performance of the endoscopic examination.

An example of a currently preferred embodiment of the device or endoscopic straightener for colonoscopy will be described below with reference to the attached figures in which:
- Fig. 1 is a perspective view of the straightener illustrated on a reduced scale;
- Fig. 2 is a plan view of the straightener in a position in which it has the protuberance pointing upwards;
- Fig 3 is a view of the straightener in profile, seen from below with respect to Fig. 2;
- Fig. 4 is a side view of the straightener, from the left with respect to Fig. 3;
- Fig. 5 is a simplified illustration of the colon;
- Fig. 5a is a diagrammatic illustration of the colon;
- Fig. 5b is a highly diagrammatic illustration of the colon in the conformation which one tends to approach in order to optimise the endoscopic examination;
- Figs 6, 7, 8, 9 and 10 illustrate various relative positions of the patient and the straightener.

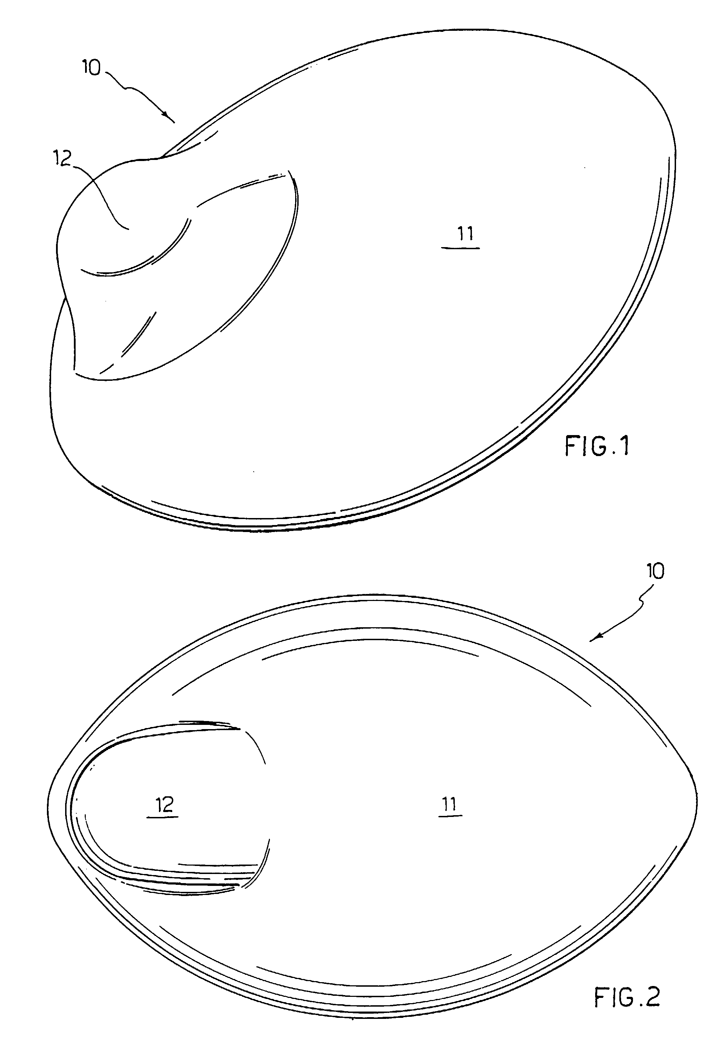

The device will first be described with reference to Figs. 1 to 4.

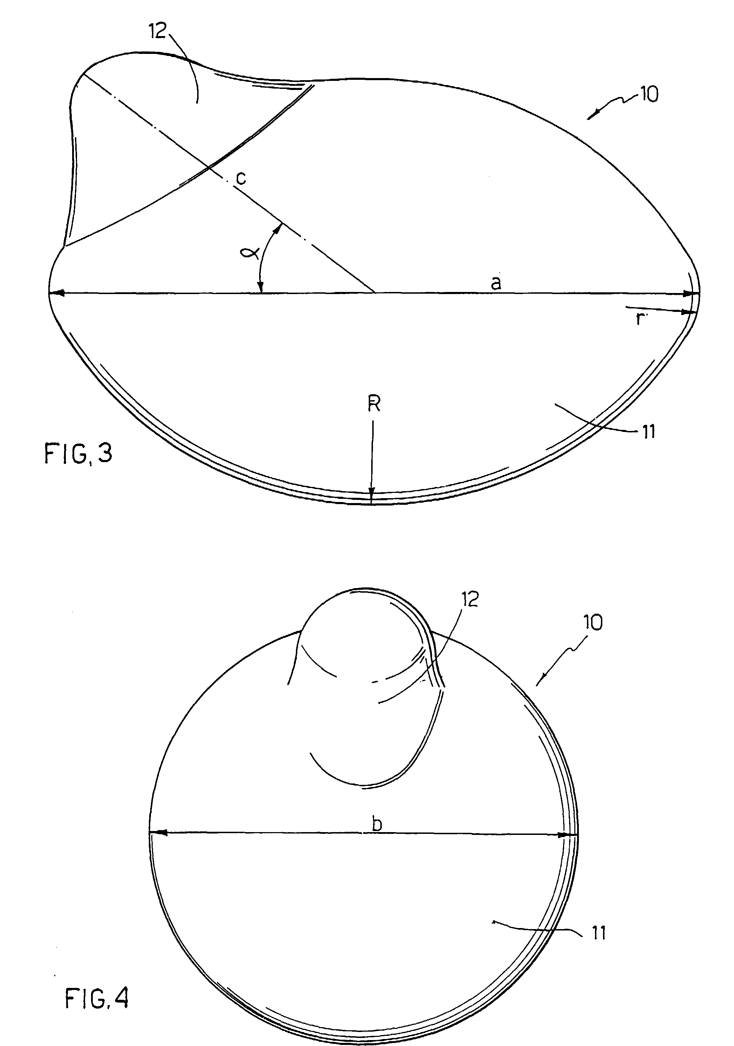

The straightener has a convex curved shape, egg-shaped or ellipsoid; hereinafter referred to generically as an ovoid shape. A major axis, indicated by a in the figures, extends from one extremity to the other, or from one end to the other, of the ovoid shape. In a section across the major axis, as can be seen in Fig. 4, the device has a substantially circular shape with a diameter, or minor axis, b. In proximity to one of the ends the device has a roundish protuberance, indicated by 12. As an indication, a position for the protuberance can be identified along an axis c inclined at 38° with respect to the longitudinal axis of the body and the protuberance projects from the body by a maximum of about 107 mm The body seen in profile preferably has a bend radius R of about 164 mm whilst the opposite ends have a bend radius r of about 32 mm. The protuberance 12 is joined to the rest of the body along surfaces that have a slightly concave shape in profile.

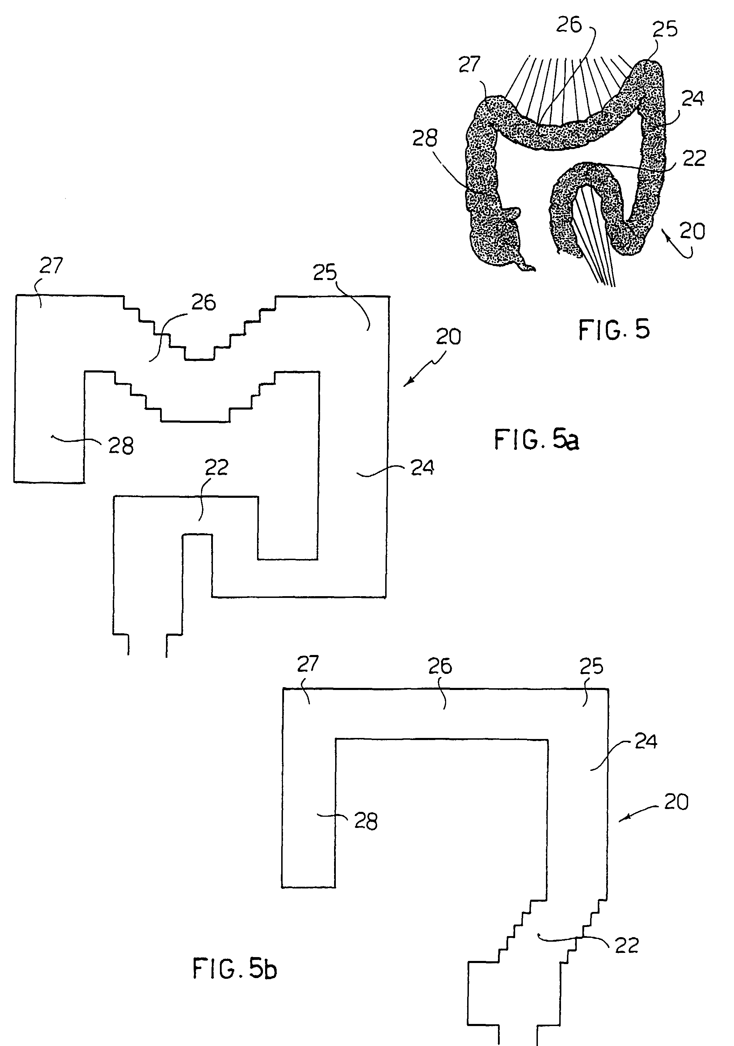

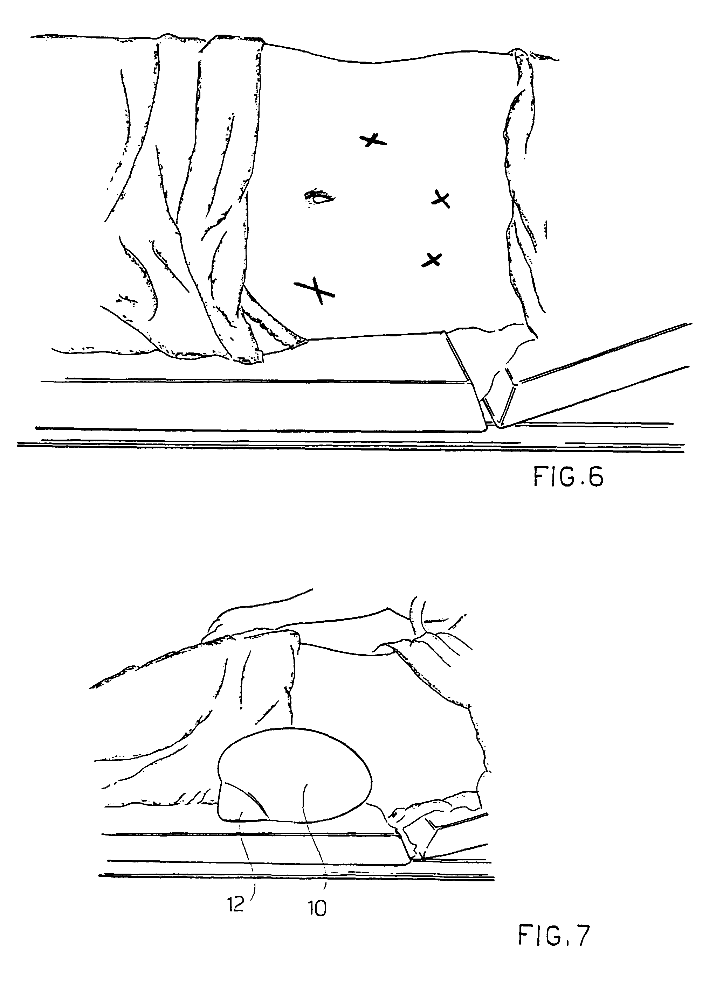

The method for use of the straightener will be described below with reference to Figs. 5 to 10 and in relation to the introduction of a colonoscope of a per se known type in the field, with UP. DOWN, RIGHT and LEFT controls for manoeuvring. To clarify, Figs. 5, 5a and 5b show the colon 20 which comprise the sigmoid, indicated by 22, the descending colon 24, the splenic flexure 25, the transverse colon 26, the hepatic flexure 27, and the ascending colon 28. The same items with the same reference numbers are indicated in the highly schematic figures 5a and 5b. In Fig. 6 the four pressure points that can be useful for introduction of a colonoscope are indicated on the anterior aspect of the trunk of a hypothetical patient. It should be noted that it is not always necessary to use all four pressure points indicated and that these could also be in a different position to that indicated.

POSITION No. 1: (Fig. 7) With the patient in left lateral decubitus on a rigid table with a soft surface the endoscopic manoeuvres to reach the proximal sigmoid are performed; the to and fro adjusting movement (jiggling) is carried out; the straightener is placed in the left iliac fossa; the protuberance is pointed towards the table and rests on it; the long axis is parallel to the descending sigmoid; the patient is turned again to a semi-prone position with the left lower limb extended and the right lower limb flexed. The patient is therefore partially resting on the straightener, and the protuberance acts as an anchor preventing any unwanted movement of the straightener. The UP-DOWN lever of the colonoscope must remain fee and the RIGHT-LEFT lever locked.

A 100-180° rotation of the colonoscope is performed, a the same time shortening the intestine; at this point the RIGHT-LEFT lever is locked and the previous position is resumed; the probe will be vertical and in line with the descending colon.

These manoeuvres attempt to smooth any convexity of the endoscope created in reaching the descending colon, subjecting the endoscopic probe, with the anti-clockwise rotation. to the fixed, uniform pressure of the straightener.

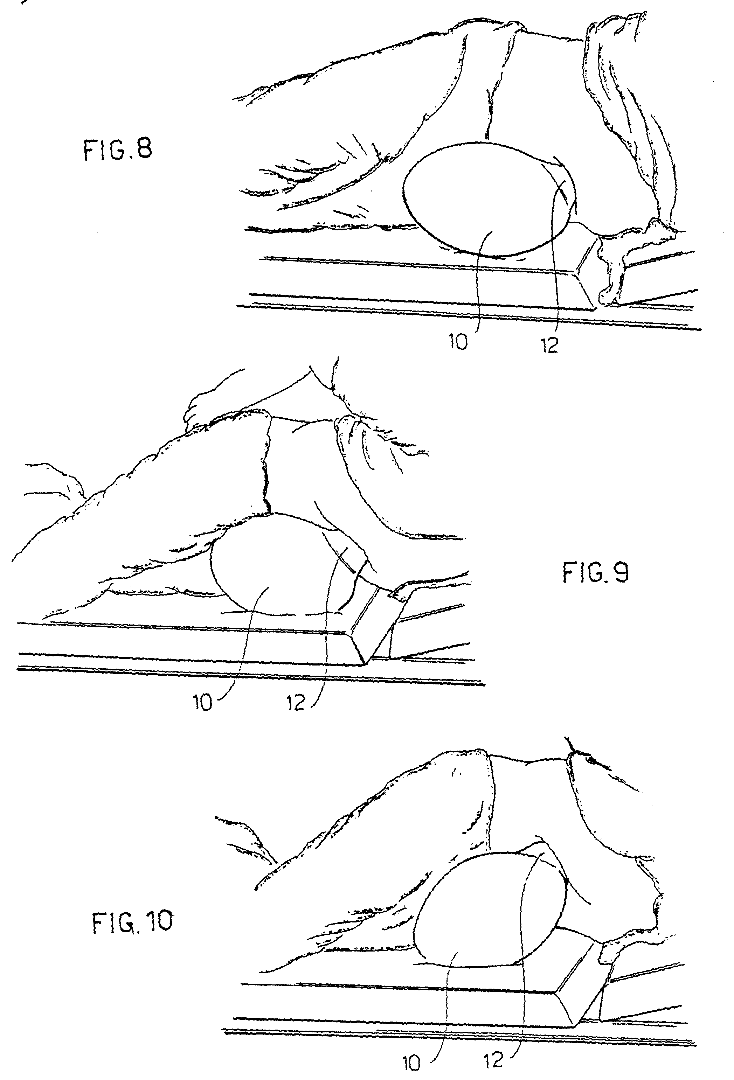

POSITION No. 2: (Fig. 8) The straightener rests on the table with the curved surface of its body; the protuberance 12 which acted as an anchor in the first position is now positioned medially to and at the bottom of the splenic flexure, that is acting as a wedge lodged in the splenic flexure and fixes the distal portion of the transverse colon, at the same time pushing it upwards, since it has a mesentery and could wave about creating wrong turns. The controlateral part of the straightener (without the protruding portion) remains on the sigmoid.

POSITION No. 3 (Fig. 9) The protuberance 12 of the straightener is now positioned vertically immediately below the xiphoid process (sternum); the convexity of the straightener body continues to press on the sigmoid. The length of the instrument (colonoscope) introduced has increased greatly and with it the friction has increased in parallel. Greater pressure is therefore necessary to advance the tip with a possible shift out of line of the instrument and a consequent traction on the mesenteries. The part of the straightener positioned on the sigmoid maintains its initial vertical position. With the straightener positioned on the transverse colon, two thrust vectors or directions are implemented, one vertical and one slanting. The vertical vector or direction is given by the rigid protuberance of the straightener; the vector slanting from the bottom upwards is given by the convexity of the straightener which pushes on the intestinal packet, supporting the transverse intestine from beneath; in fact these two force vectors form a fixed fork in the hollow of which is positioned the transverse intestine in which the endoscope slides. The transverse intestine will thus be supported and directed by the fork created by the protuberance and the convexity of the straightener. If the manoeuvre does not succeed, the protuberance is lowered beneath the umbilicus always maintaining it on a median line, in that the failure of the previous position indicates an excessive ptosis of the bowel.

POSITION No. 4: (Fig. 10) The straightener is placed with the convexity on the sigmoid and the protuberance vertically positioned medially to and at the bottom of the right flexure (hepatic) supporting the right transverse colon and keeping it in line.

The advantages are considerable; they are:
1) extremely rapid execution; as little as a few minutes for examinations that required more than 20 minutes of half an hour;
2) even inexperienced operators can carry out pancolonoscopies;
3) less wear on the endoscope;
4) less discomfort for the patients with the possibility of performing this examination even in patients at risk;
5) endoscopic manoeuvres can be planned; therefore the examination is easier to learn and to teach;
6) because of the specificity of its shape and therefore of its function, the straightener can exert a specific and non-specific compression at the same operating time, effecting a manoeuvre that no nurse could carry out;
7) the compression is actually applied by the patient with his/her own weight;
8) the operator, with his clinical experience, can modify the application points of the thrust vectors as he pleases according to his own personal views or experience;
9) the same straightener can easily be used to carry out compression manoeuvres during radiological and cystoscopic examinations.

## Claims

1. A external straightener **(10)** for colonoscopy, characterised in that it comprises a body **(11)** having a convex curved shape, with a major axis **(a)** and a minor axis **(b)**, said body being provided, in proximity to one of its ends, with a roundish protuberance **(12)**, joined to the body.

2. A straightener according to claim 1, characterised in that the shape of the body **(11)** is one of the following: ovoid, ellipsoid, or as a rugby ball.

3. An external straightener for colonoscopy according to claim 1, characterised in that the major axis **(a)** of said body **(11)** has a length of about 300 mm. and the minor axis **(b)** of said body (11) has a length of about 200 mm.

4. An external straightener for colonoscopy according to claim 1, characterised in that the major axis **(a)** of the body is between about 50 mm and 400 mm, preferably 292 mm, and the median cross section of body is circular with a diameter **(b)** between 50 and 250 mm, preferably about 193 mm.

5. An external straightener for colonoscopy according to claim 1, characterised in that the surface of the protuberance **(12)** farthest from the longitudinal axis **(a)** of the body is at a distance of about 107 mm from it **(a)**.

6. An external straightener for colonoscopy according to any one of the preceding claims, characterised in that a major axis of said protuberance has a length of about 140 mm., and a minor axis of said protuberance has a length of about 110 mm.

7. An external straightener for colonoscopy according to any one of the preceding claims, characterised in that it is made of rubber, or PVC, natural latex or similar.

8. An external straightener for colonoscopy according to any one of the preceding claims, characterised in that it has a weight of about 3.5 kg.

9. An external straightener for colonoscopy according to any one of the preceding claims, characterised in that the body has a convex curved surface with a radius **(R)** of about 164 mm.

10. An external straightener for colonoscopy according to any one of the preceding claims, characterised in that the protuberance (12) is joined to the body (11) by concave surfaces.

## Patentansprüche

1. Äußerer Geraderücker (10) für Koloskopie, dadurch gekennzeichnet, daß er einen Körper (11) mit konvex gebogener Form mit einer größeren Achse (a) und einer kleineren Achse (b) umfaßt, wobei dieser Körper in der Nähe einer seiner Pole mit einer Protuberanz (12) rundlicher Form versehen ist, die mit dem Körper verbunden ist.

2. Geraderücker nach Anspruch 1, dadurch gekennzeichnet, daß die Form des Körpers (11) eine der folgenden ist: eiförmig, oder ellipsoid oder die Form eines Rugbyballs.

3. Äußerer Geraderücker für Koloskopie nach Anspruch 1, dadurch gekennzeichnet, daß die größere Achse (a) des besagten Körpers (11) eine Länge von etwa 300 mm und die kleinere Achse (b) des besagten Körpers (11) eine Länge von etwa 200 mm aufweist.

4. Äußerer Geraderücker für Koloskopie nach Anspruch 1, dadurch gekennzeichnet, daß die größere Achse (a) des Körpers von etwa 50 mm. bis 400 mm, vorzugsweise 292 mm beträgt, und der mittlere Querschnitt des Körpers rund ist, mit einem Durchmesser (b) von 50 bis 250, vorzugsweise etwa 193 mm.

5. Äußerer Geraderücker für Koloskopie nach Anspruch 1, dadurch gekennzeichnet, daß die Protuberanz (12) die Oberfläche mit größtem Abstand von der Längsachse (a) des Körpers bei etwa 107 mm davon (a) hat.

6. Äußerer Geraderücker für Koloskopie nach einem beliebigen der vorausgegangenen Ansprüche, dadurch gekennzeichnet, daß eine größere Achse der besagten Protuberanz eine Länge von etwa 140 mm und eine kleinere Achse der besagten Protuberanz eine Länge von etwa 110 mm aufweist.

7. Äußerer Geraderücker für Koloskopie nach einem beliebigen der vorausgegangenen Ansprüche, dadurch gekennzeichnet, daß er aus Gummi, oder PVC, natürlichem Latex oder ähnlichem Material besteht.

8. Äußerer Geraderücker für Koloskopie nach einem beliebigen der vorausgegangenen Ansprüche, dadurch gekennzeichnet, daß er ein Gewicht von etwa 3,5 kg aufweist.

9. Äußerer Geraderücker für Koloskopie nach einem beliebigen der vorausgegangenen Ansprüche, dadurch gekennzeichnet, daß der Körper eine konvex gebogene Oberfläche mit einem Radius (R) von etwa 164 mm aufweist.

10. Äußerer Geraderücker für Koloskopie nach einem beliebigen der vorausgegangenen Ansprüche, dadurch gekennzeichnet, daß die Protuberanz (12) durch konkave Oberflächen mit dem Körper (11) verbunden ist.

## Revendications

1. Redresseur externe (10) pour côlonoscopie caractérisé par le fait qu'il comprend un corps (11) de forme courbe convexe, avec un axe majeur (a) et un axe mineur (b), ledit corps étant pourvu, près de l'un de ses pôles, d'une protubérance (12) de forme arrondie, raccordée au corps.

2. Redresseur selon la revendication 1 caractérisé par le fait que la forme du corps (11) est l'une des suivantes : ovoïdale, en ellipse ou en ballon de rugby.

3. Redresseur externe pour côlonoscopie selon la revendication 1 caractérisé par le fait que l'axe majeur (a) dudit corps (11) a une longueur de 300 mm environ et l'axe mineur (b) dudit corps (11) a une longueur de 200 mm environ.

4. Redresseur externe pour côlonoscopie selon la revendication 1 caractérisé par le fait que l'axe majeur (a) du corps est de 50 à 400 mm environ, de préférence de 292 mm, la section transversale médiane du corps est circulaire avec un diamètre (b) de 50 à 250, de préférence de 193 mm environ.

5. Redresseur externe pour côlonoscopie selon la revendication 1 caractérisé par le fait que la protubérance (12) a la surface qui se trouve la plus distante de l'axe longitudinal (a) du corps à environ 107 mm de celui-ci (a).

6. Redresseur externe pour côlonoscopie selon n'importe laquelle des revendications précédentes caractérisé par le fait qu'un axe majeur de ladite protubérance a une longueur de 140 mm environ, et un axe mineur de ladite protubérance a une longueur de 110 mm environ.

7. Redresseur externe pour côlonoscopie selon n'importe laquelle des revendications précédentes caractérisé par le fait d'être réalisé en caoutchouc, ou PVC, latex naturel ou autres matériaux semblables.

8. Redresseur externe pour côlonoscopie selon n'importe laquelle des revendications précédentes caractérisé par le fait de avoir un poids de 3,5 kg environ.

9. Redresseur externe pour côlonoscopie selon n'importe laquelle des revendications précédentes caractérisé par le fait que le corps présente une superficie courbe convexe avec un rayon (R) de 164 mm environ.

10. Redresseur externe pour côlonoscopie selon n'importe laquelle des revendications précédentes caractérisé par le fait que la protubérance (12) est raccordée au corps (11) par des surfaces concaves.
